# EUROPEAN PATENT SPECIFICATION

(11) **EP 2 923 689 B1**
(45) Date of publication and mention of the grant of the patent: **18.03.2020**
(21) Application number: 14001147.9
(22) Date of filing: 27.03.2014
(51) Int. Cl.: A61K 6/083, A61K 6/00

(54) **Dental composition**
Dentale Zusammensetzung
Composition dentaire

(43) Date of publication of application: 30.09.2015
(73) Proprietor: DENTSPLY DETREY GmbH, 78467 Konstanz (DE)
(72) Inventor: Klee, Joachim, 78315 Radolfzell (DE); Maier, Maximilian, 40213 Düsseldorf (DE); Lalevée, Jacques, 68200 Mulhouse (FR); Fouassier, Jean Pierre, 68590 St. Hippolyte (FR); Morlet-Savary, Fabrice, 68120 Pfastatt (FR)
(74) Representative: Dietz, Mirko

(56) References cited:
- WO-A1-2013/153166
- US-A1- 2009 239 967
- J. LALEVÉE ET AL: "Silanes as New Highly Efficient Co-initiators for Radical Polymerization in Aerated Media", MACROMOLECULES, vol. 41, no. 6, 1 March 2008 (2008-03-01), pages 2003-2010, XP055139430, ISSN: 0024-9297, DOI: 10.1021/ma702301x
- MOHAMAD EL-ROZ ET AL: "Mechanistic Investigation of the Silane, Germane, and Stannane Behavior When Incorporated in Type I and Type II Photoinitiators of Polymerization in Aerated Media", MACROMOLECULES, vol. 42, no. 22, 24 November 2009 (2009-11-24), pages 8725-8732, XP055139433, ISSN: 0024-9297, DOI: 10.1021/ma9017313

## Description

### Field of the Invention

The present invention relates to a dental composition comprising a specific polymerization initiator system. Moreover, the present invention relates to the use of the specific polymerization initiator system for the preparation of a dental composition.

A polymerization initiator system of the present invention may be used for providing dental compositions having excellent mechanical properties, handling and storage properties, biocompatibility as well as aesthetic properties.

### Background of the Invention

WO 2013/153166 discloses acyl germanes for use as initiators in dental compositions. The restoration of teeth commonly involves a light curable dental composition containing free-radically polymerizable resins. Light curing of a dental composition involves a photoinitiator system generating free radicals upon exposure to visible light. Free radicals may be typically produced by either of two pathways:
(1) the photoinitiator compound undergoes excitation by energy absorption with subsequent decomposition of the compound into one or more radicals (Norrish type I), or
(2) the photoinitiator compound undergoes excitation and the excited photoinitiator compound interacts with a second compound by either energy transfer or a redox reaction to form free radicals from any of the compounds (Norrish type II).

In order for a photoinitiator to be useful for use in a dental composition, the quantum yields indicating the conversion of light radiation to radical formation needs to be high since absorption or shielding of light by further components of the dental composition limit the amount of energy available for absorption by the photoinitiators. Accordingly, only 70 percent conversion of the polymerizable groups may be expected in a polymerization of a typical dental composition, whereby the mechanical strength of the polymerized dental composition is rather limited band unreacted monomers may leach out from the polymerized dental composition. The leaching monomers may have detrimental effects. In order to alleviate this problem, multifunctional monomers are frequently used which are more likely to be included in the polymer network.

In addition, photoinitiators are required to have a high solubility, thermal stability, and storage stability when incorporated into a dental composition.

Finally, given that dental compositions usually contain (meth)acrylate or (meth)acrylamide monomers, free radical photocuring may be inhibited by the presence of oxygen. Oxygen inhibition is due to the rapid reaction of propagating radicals with oxygen molecules to yield peroxyl radicals which are not as reactive towards carbon-carbon unsaturated double bonds and therefore do not initiate or participate in any photopolymerization reaction. Oxygen inhibition may lead to premature chain termination and, therefore, incomplete photocuring.

Accordingly, the polymerization initiator system has a critical influence on the quality of the dental material.

Photopolymerizable dental compositions usually contain a polymerization initiator system comprising a combination of a sensitizer and a hydrogen donating agent such as alpha-diketone/amine. A preferred alpha-diketone sensitizer is camphor quinone (CQ). CQ may absorb visible light and form a photoexcitation complex (CQ*-amine exciplex) with a tertiary amine, e.g. ethyl 4-dimethylaminobenzoat (EDMAB).

In the course of the polymerization reaction of the dental composition, the sensitizer is required to become efficiently discoloured in order to avoid undesired coloration of the cured dental composition. Moreover, the polymerization initiator system is required to provide a high reactivity when activated by light and high storage stability even when stored in an acidic medium. Furthermore, the polymerization initiator system is required to be soluble in the dental composition.

Moreover, ternary photoinitiator systems are known wherein a sensitizer (e.g. CQ), is used in combination with an amine (e.g. EDMAB), and an iodonium salt (i.e. diphenyliodonium hexafluoroantimonate).

The presence of amines in acrylate-containing compositions can cause yellowing in the resulting photocured composition, create undesirable odors, and soften the cured composition because of chain transfer reactions and therefore, frequently requires the use of stabilizers. Moreover, the use of aromatic amines such as EDMAB gives rise to toxicological concerns.

Furthermore, it is desirable that the light activating the photoinitiator system has a longer wavelength than 350 nm in order to avoid damage in the patients gingival tissue during polymerization of the dental composition. Accordingly, the photoinitiator system is required to contain a chromophoric group efficiently absorbing light of the desired wavelength in a range from 400 to 800 nm. However, an increase of the absorption coefficient of the photoinitiator system increases the color intensity of the photoinitiator system and thereby the color intensity of the dental composition as a whole. Accordingly, it is necessary that the chromophoric groups are efficiently destroyed during polymerization so that the color intensity of the initiator system disappears in the polymerized dental composition.

### Summary of the Invention

It is the problem of the present invention to provide a dental composition comprising a polymerization initiator system, which has excellent mechanical properties after curing, excellent handling and storage properties before curing, and excellent biocompatibility as well as aesthetic properties when applied to a patient.

Moreover, it is the problem of the present invention to provide a specific polymerization initiator system which may be used for the preparation of a dental composition having excellent mechanical properties after curing, excellent handling and storage properties before curing, and excellent biocompatibility as well as aesthetic properties when applied to a patient.

According to a first aspect, the present invention provides a dental composition comprising
(i) 5 to 80 percent by weight based on the total weight of the composition of a polymerizable matrix containing polymerizable monomers;
(ii) a polymerization initiator system containing
   (a) an alpha-diketone photoinitiator compound having a light absorption maximum in the range from 300 to 500 nm; and
   (b) a coinitiator compound of the following formula (I):

      A-H (I)

      wherein A is a moiety of the following formula (II)

      R¹R²R³X- (II)

      wherein
      X represents Si, Ge, or Sn and
      R¹ represents a hydrogen atom, an organic moiety or a different moiety A;
      R² and R³ which are independent from each other, represent an organic moiety, wherein the dental composition is selected from dental adhesives containing monomers dissolved in one or more solvents and dental composites containing monomers wherein one or more particulate fillers are dispersed.

According to a second aspect, the present invention provides a use of a polymerization initiator system containing
(a) an alpha-diketone photoinitiator compound having a light absorption maximum in the range from 300 to 500 nm; and
(b) a coinitiator compound of the following formula (I):

   A-H (I)

   wherein A is a tetravalent moiety of the following formula (II)

   R¹R²R³X- (II)

   wherein
   - X: represents Si, Ge, or Sn and
   - R¹: represents a hydrogen atom, an organic moiety or a different moiety A;
   - R² and R³: which are independent from each other represent an organic moiety,
for the preparation of a dental composition selected from dental adhesives containing monomers dissolved in one or more solvents and dental composites containing monomers wherein one or more particulate fillers are dispersed.

According to the present invention, it has been found that a polymerization initiator system containing an alpha-diketone and a specific coinitiator compound of the formula (I) may be used to provide a dental composition exhibiting excellent mechanical properties after curing, excellent handling and storage properties before curing, and excellent biocompatibility as well as aesthetic properties when applied to a patient.

The specific polymerization initiator systems according to the present invention may be used for replacing conventional alpha-diketone/amine polymerization initiators such as CQ/EDMAB for dental compositions. Surprisingly, dental compositions comprising the initiator system of the present invention show improved mechanical properties compared to the corresponding compositions comprising alpha-diketone/amine polymerization initiators.

The dental composition of the present invention may be advantageously used as tooth-colored dental restorative composition where it is essential to avoid undesired discoloration after curing of the composition so that the final restoration retains a natural, tooth-like appearance that persistently matches the colour of surrounding teeth.

### Detailed Description of Preferred Embodiments

The term "visible light" refers to light having a wavelength of 400 to 800 nanometers (nm).

A "monomer" is compound having polymerizable groups, preferably carbon-carbon double bonds (including (meth)acrylate or (meth)acrylamide groups)) which can be free-radically polymerized to oligomers or polymers.

An "initiator or initiator system" is a substance or mixture which is able to start the free-radical polymerization of a monomer.

A "dental composition" is a composition containing monomers, which can be used in the dental field for the treatment of a patient. Dental compositions may be dental adhesives containing monomers dissolved in one or more solvents. Dental compositions may be dental composites containing monomers wherein one or more particulate fillers are dispersed. A dental composition should be biocompatible in the sense that toxic components are prevented from migrating out of the cured composition.

The present invention provides a dental composition. In case the dental composition is a dental composite, the inventive dental composite can be characterized by at least one of the following features after hardening:

| | |
|---|---|
| Flexural strength: | at least 80 or at least 100 or at least 110 MPa determined according to ISO 4049. |
| Compressive strength: | at least 250 or at least 280 or at least 320 MPa, determined according to ISO 9917 using cubic specimen (dimensions 3 mm^{∗}3 mm^{∗}5 mm), |
| E-modulus: | at least 7 or at least 8 or at least 9 GPa determined according to ISO 4049, |
| Depth of cure: | at least about 2.00 mm, (measured values of depth of cure in a metal mold according to ISO 4049) |

In case the dental composition is a dental adhesive, the inventive dental adhesive can be characterized by at least one of the following features after hardening:

| | |
|---|---|
| Adhesive strength: | at least 10 or at least 14 or at least 20 MPa determined according to ISO 1994-ISO TR 11405. |

The dental composition comprises 5 to 80 percent by weight based on the total weight of the composition of a polymerizable matrix containing polymerizable monomers. A dental composite typically contains 5 to 40 percent by weight, preferably 10 to 30 percent by weight based on the total weight of the composition of a polymerizable matrix containing polymerizable monomers. A dental adhesive typically contains 20 to 80 percent by weight, preferably 25 to 50 percent by weight based on the total weight of the composition of a polymerizable matrix containing polymerizable monomers.

The polymerizable matrix may contain polymerizable monomers selected from mono-, bi-, tri- or polyfunctional monomers. The polymerizable monomers may be selected from methyl (meth)acrylate, isobutyl (meth)acrylate, benzyl (meth)acrylate, lauryl (meth)acrylate, 2-(N,N-dimethylamino)ethyl (meth)acrylate, 2,3-dibromopropyl (meth)acrylate, 2-hydroxyethyl (meth)acrylate, 6-hydroxyhexyl (meth)acrylate, 10-hydroxydecyl (meth)acrylate, propylene glycol mono(meth)acrylate, glycerol mono(meth)acrylate, erythritol mono(meth)acrylate, N-methylol (meth)acrylamide, N-hydroxyethyl (meth)acrylamide, N-(dihydroxyethyl)(meth)acrylamide, (meth)acryloyloxydodecylpyridinium bromide, (meth)acryloyloxydodecylpyridinium chloride, (meth)acryloyloxyhexadeylpyridinium chloride, (meth)acryloyloxydecylammonium chloride, ethylene glycol di(meth)acrylate, triethylene glycol di(meth)acrylate, propylene glycol di(meth)acrylate, neopentyl glycol di(meth)acylate, 1,6-hexanediol di(meth)acrylate, 1,10-decanediol di(meth)acrylate, bisphenol A diglycidyl (meth)acrylate (2,2-bis[4-[3-(meth)acryloyloxy-2-hydroxypropoxy]phenyl]propane, commonly known as "BisGMA"), 2,2-bis[4-(meth)acryloyloxyethoxyphenyl]propane, 2,2-bis[4-(meth)acryloyloxyolyothoxyphenyl]propane, 2,2-bis[4-[3-((meth)acryloyloxy-2-hydroxypropoxy]phenyl]propane, 1,2-bis[3-(meth)acryloyloxy-2-hydroxypropoxylethane, pentaerythritol di(meth)acrylate, [2,2,4-trimethylhexamethylenebis(2-carbamoyloxyethyl)] dimethacrylate (commonly known as "UDMA trimethylolpropane tri(meth)acrylate, trimethylolethane tri(meth)acrylate, tetramethylolmethane tri(meth)acrylate, pentaerythritol tetra(meth)acrylate, dipentaerythritol hexa(meth)acrylate, N,N'-(2,2,4-trimethylhexamethylene)bis[2-(aminocarbonyloxy)propane-1,3-diol] tetramethacrylate, and 1,7-diacryloyloxy-2,2,6,6-tetraacryloyloxymethyl-4-oxyheptane.

The polymerizable matrix may also contain polymerizable monomers selected from N,N'-diethyl-1,3-bis(acrylamido)-propane, 1,3-bis(methacrylamido)-propane, 1,4-bis(acrylamido)-butane, and 1,4-bis(acryloyl)-piperazine, N-ethylacrylamide, N,N-dimethylacrylamide, N-methyl-N-(2-hydroxyethyl)acrylamide, N-ethylmethacrylamide, 2,2,4-trimethylhexamethylene diisocyanate or isophorone diisocyanate, methylene or ethylene bisacrylamide.

The dental composition further comprises a specific polymerization initiator system. The polymerization initiator system consisting of an alpha-diketone photoinitiator compound and a coinitiator compound may be contained in the dental composition in an amount from 0.01 to 10 percent by weight, more preferably 0.5 to 7 percent by weight, still more preferably 1 to 5 percent based on the total weight of the dental composition.

The polymerization initiator system contains an alpha-diketone photoinitiator compound having a light absorption maximum in the range from 300 to 500 nm. The alpha-diketone photoinitiator is a sensitizer capable of absorbing visible light and forming a photoexcitation complex with a hydrogen donating compound. The alpha-diketone photoinitiator compound may be selected from camphorquinone, 1,2-diphenylethane-1,2-dione (benzil), 1,2-cyclohexanedione, 2,3-pentanedione, 2,3-hexanedione, 3,4-hexanedione, 2,3-heptanedione, 3,4-heptanedione glyoxal, biacetyl, 3,3,6,6-tetramethylcyclohexanedione, 3,3,7,7-tetramethyl-1,2-cycloheptanedione, 3,3,8,8-tetramethyl-1,2-cyclooctanedione; 3,3,18,18-tetramethyl-1,2-cyclooctadecanedione; dipivaloyl; furil, hydroxybenzil, 2,3-butanedione, 2,3 -octanedione, 4,5-octanedione, and 1-phenyl-1,2-propanedione. Camphorquinone is the most preferred alpha-diketone photoinitiator.

According to a preferred embodiment, the polymerizable matrix contains the alpha-diketone photoinitiator in an amount from 0.05 to 5 mole percent.

The polymerization initiator system further contains a coinitiator compound of the following formula (I):

A-H (I)

The coinitiator compound is a metal hydride. The metal hydride of formula (I) may react as a hydrogen donating agent in a photoexcitation complex with the alpha-diketone sensitizer. Accordingly, when alpha-diketone absorbs visible light and forms an exciplex with the metal hydride of formula (I), a hydrogen transfer may take place from the metal hydride to the alpha-diketone compound whereby the coinitiator of is transformed into a radical specifies capable of facilitating the polymerization reaction.

In formula (I), A is a moiety of the following formula (II)

R¹R²R³X- (II)

In formula (II), X represents Si, Ge, or Sn. Preferably, X represents Si or Ge. According to a specific embodiment, the coinitiator compound is a silane compound. According to a further specific embodiment, the coinitiator compound is a germane compound.

In formula (II), R¹ may be a hydrogen atom, an organic moiety or a different moiety A. When R¹ is a hydrogen atom, then the coinitiator compound contains two metal hydride bonds (X-H). In case R¹ is a hydrogen atom, the X is Si.

When R¹ is an organic moiety, R¹ is preferably an aromatic, an aliphatic or an alicyclic group. An aromatic group may be a phenyl group. The phenyl group may be substituted by one or more straight chain or branched alkyl groups having 1 to 6 carbon atoms, alicyclic groups having 3 to 6 carbon atoms, halogen atoms, hydroxyl groups, or amino groups. The aliphatic group may be a straight chain or branched alkyl groups having 1 to 6 carbon atoms which may be substituted by one or more aromatic groups, alicyclic groups having 3 to 6 carbon atoms, halogen atoms, hydroxyl groups or amino groups. An alicyclic group may be a group having 3 to 6 carbon atoms which may be substituted by one or more aromatic groups, aliphatic groups, halogen atoms, hydroxyl groups or amino groups.

When R¹ is a different moiety A, the coinitiator compound of the formula (I) contains a metal-metal bond. In case two moieties A are present, then each X, R¹, R² and R³ may be the same or different and independently has the meaning as defined by the present invention.

R² and R³ which are independent from each other, represent an organic moiety. An organic group may be an aromatic, an aliphatic or an alicyclic group. An aromatic group may be a phenyl group. The phenyl group may be substituted by one or more straight chain or branched alkyl groups having 1 to 6 carbon atoms, alicyclic groups having 3 to 6 carbon atoms, halogen atoms, hydroxyl groups, or amino groups. The aliphatic group may be a straight chain or branched alkyl groups having 1 to 6 carbon atoms which may be substituted by one or more aromatic groups, alicyclic groups having 3 to 6 carbon atoms, halogen atoms, hydroxyl groups or amino groups. An alicyclic group may be a group having 3 to 6 carbon atoms which may be substituted by one or more aromatic groups, aliphatic groups, halogen atoms, hydroxyl groups or amino groups.

According to a preferred embodiment, R¹, R², and R³ in the coinitiator compound of formula (I) are the same and represent an aliphatic, an aromatic or an alicyclic hydrocarbon group.

According to a preferred embodiment, the coinitiator compound of formula (I) is a compound of the following formula:

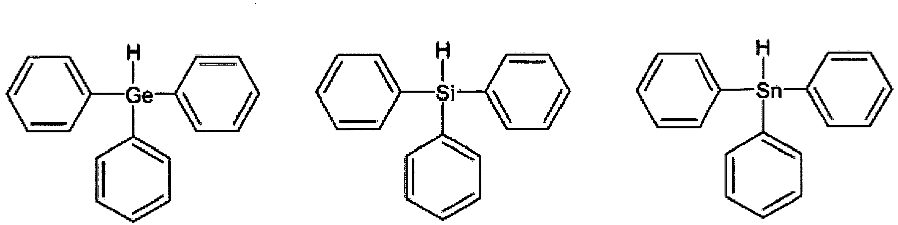

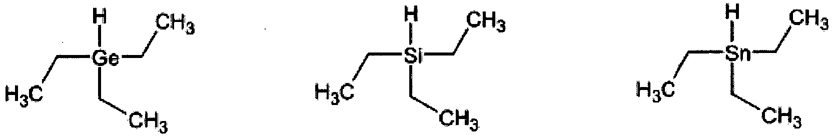

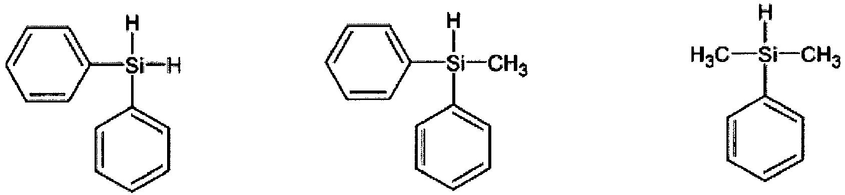

According to a preferred embodiment, the polymerizable matrix contains the coinitiator compound in an amount from 0.05 to 5 percent by weight based on the total weight of the composition.

The polymerization initiator system of the dental composition according to the present invention may further comprise
(1) a iodonium compound of the following formula (III):

   R⁴-I⁺-R⁵ Y⁻ (III)

   wherein
   - R⁴ and R⁵: which are independent from each other represent an organic moiety, and
   - Y⁻: is an anion.

Preferably, R⁴ and R⁵ represent an aromatic, an aliphatic or an alicyclic group. An aromatic group may be a phenyl group. The phenyl group may be substituted by one or more straight chain or branched alkyl groups having 1 to 6 carbon atoms, straight chain or branched alkoxy groups having 1 to 6 carbon atoms, aromatic groups such as aryl groups or aryloxy groups, alicyclic groups having 3 to 6 carbon atoms, halogen atoms, hydroxyl groups, or amino groups. The aliphatic group may be a straight chain or branched alkyl groups having 1 to 6 carbon atoms which may be substituted by one or more aromatic groups, alicyclic groups having 3 to 6 carbon atoms, halogen atoms, hydroxyl groups or amino groups. An alicyclic group may be a group having 3 to 6 carbon atoms which may be substituted by one or more aromatic groups, aliphatic groups, halogen atoms, hydroxyl groups or amino groups.

According to a preferred embodiment, the iodonium salt is a diaryl iodonium salt. Examples of useful diaryl iodonium salt include (4-methylphenyl)[4-(2-methylpropyl) phenyl] iodonium hexafluoroantimonate, include (4-methylphenyl)[4-(2-methylpropyl) phenyl] iodonium tetrafluoroborate, diphenyliodonium tetrafluoroborate, di(4-methylphenyl)iodonium tetrafluoroborate, phenyl-4-methylphenyliodonium tetrafluoroborate, di(4-heptylphenyl)iodonium tetrafluoroborate, di(3-nitrophenyl)iodonium hexafluorophosphate, di(4-chlorophenyl)iodonium hexafluorophosphate, di(naphthyl)iodonium tetrafluoroborate, di(4-trifluoromethylphenyl)iodonium tetrafluoroborate, diphenyliodonium hexafluorophosphate, di(4-methylphenyl)iodonium hexafluorophosphate; diphenyliodonium hexafluoroarsenate, di(4-phenoxyphenyl)iodonium tetrafluoroborat, phenyl-2-thienyliodonium hexafluorophosphate, 3,5-dimethylpyrazolyl-4-phenyliodonium hexafluorophosphate, diphenyliodonium hexafluoroantimonate, 2,2'-diphenyliodonium tetrafluoroborate, di(2,4-dichlorophenyl)iodonium hexafluorophosphate, di(4-bromophenyl)iodonium hexafluorophosphate, di(4-methoxyphenyl)iodonium hexafluorophosphate, di(3-carboxyphenyl)iodonium hexafluorophosphate, di(3-methoxycarbonylphenyl)iodonium hexafluorophosphate, di(3-methoxysulfonylphenyl)iodonium hexafluorophosphate, di(4-acetamidophenyl)iodonium hexafluorophosphate, di(2-benzothienyl)iodonium hexafluorophosphate, and diphenyliodonium hexafluoroantimonate.

Of the aromatic iodonium complex salts which are suitable for use in the compositions of the invention, include diaryliodonium hexafluorophosphate, diaryliodonium hexafluoroantimonate, (4-methylphenyl)[4-(2-methylpropyl) phenyl] iodonium hexafluoroantimonate, include (4-methylphenyl)[4-(2-methylpropyl) phenyl] iodonium tetrafluoroborate, 4-octyloxyphenyl phenyliodonium hexafluoroantimonate, 4-(2-hydroxytetradecyloxyphenyl)phenyliodonium hexafluoroantimonate, and 4-(1-methylethyl)phenyl 4-methylphenyliodonium tetrakis(pentafluorophenyl)borate.

According to a preferred embodiment, the polymerizable matrix contains the diphenyl iodonium compound in an amount from 0.001 to 2 percent by weight based on the total weight of the composition.

The polymerization initiator system of the dental composition according to the present invention may further comprise
(2) a sulfonium compound of the following formula (IV):

R⁶R⁷R⁸S⁺Y⁻ (IV)

wherein
- R⁶, R⁷ and R⁸: which are independent from each other, represent an organic moiety or wherein any two of R⁶, R⁷ and R⁸ form a cyclic structure together with the sulphur atom to which they are bound,and
- Y⁻: is an anion.

A preferred compound of the formula (IV) is S-(phenyl)thianthrenium hexafluorophosphate of the following formula:

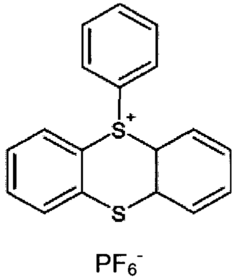

The polymerization initiator system of the dental composition according to the present invention may further comprise
(3) a phosphonium compound of the following formula (V):

R⁹R¹⁰R¹¹R¹²P⁺Y⁻ (V)

wherein
- R⁹, R¹⁰, R¹¹, and R¹²: which are independent from each other, represent an organic moiety, and
- Y⁻: is an anion.

The polymerization initiator system of the dental composition according to the present invention may further comprise
(4) a pyridinium salt.

The pyridinium salt may be a compound of the following formula (VI a-c): 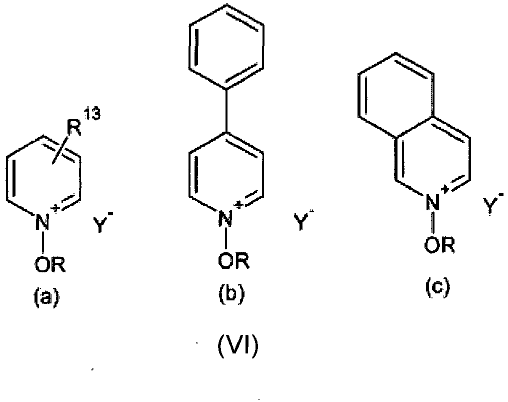 wherein
- R: represents a straight chain or branched alkyl group having 1 to 8 carbon atoms,
- R¹³: represents a straight chain or branched alkyl group having 1 to 8 carbon atoms, and
- Y⁻: is an anion.

In a salt of a compound of any one of formula (IV) to (VI), the anion may be an anion selected from halogenides such as chloride, bromide and iodide, hexafluorophosphate, tetrafluoroborate, tetraphenylborate, hexafluoroantimonate, trifluoromethylsulfonate, and hexafluoroarsenate.

The dental composition of the present invention may be a dental adhesive composition, a dental pit and fissure sealer, a dental composite, and a root canal filling composition. A dental composite may be a flowable dental composite, a universal dental composite, or packable dental composite. The dental composition is preferably selected from a dental adhesive composition, a dental pit and fissure sealer, a dental composite, and a root canal filling composition.

The dental composite of the present invention may comprise particulate filler. Particulate filler is a powdered metal oxide or hydroxide, mineral silicate, or ion leachable glass or ceramic, or mixtures thereof. Examples of particulate fillers may be selected from fillers currently used in dental restorative compositions.

The particulate filler may have a unimodal or polymodal (e.g., bimodal) particle size distribution. The particulate filler can be an inorganic material. It can also be a crosslinked organic material that is insoluble in the polymerizable resin, and is optionally filled with inorganic filler. The particulate filler can be radiopaque, radiolucent or non-radiopaque.

Examples of suitable particulate inorganic fillers are naturally-occurring or synthetic materials such as quartz, nitrides such as silicon nitride, glasses derived from, for example Ce, Sb, Sn, Zr, Sr, Ba and Al, colloidal silica, feldspar, borosilicate glass, kaolin, talc, titania, and zinc glass, and submicron silica particles such as pyrogenic silicas. Examples of suitable particulate organic filler particles include filled or unfilled pulverized polycarbonates or polyepoxides.

Preferably the surface of the filler particles is treated with a coupling agent in order to enhance the bond between the particulate filler and the matrix. The use of suitable coupling agents include gamma-methacryloxypropyltrimethoxysilane, gamma-mercaptopropyltriethoxysilane and gamma-aminopropyltrimethoxysilane.

The particulate filler usually has an average particle size from 0.005 to 100 µm, preferably from 0.01 to 40 µm as measured using, for example, by electron microscopy or by using a conventional laser diffraction particle sizing method as embodied by a MALVERN Mastersizer S or MALVERN Mastersizer 2000 apparatus.

A dental composition of the present invention may contain nano-scale particles. As the nano-scale particles in the present invention, any known nano-scale particles used in dental compositions may be used without any limitation. Preferable examples of the nano-scale particles include particles of inorganic oxides such as silica, alumina, titania, zirconia, particles of composite oxides of any of these oxides, and particles of calcium phosphate, hydroxyapatite, yttrium fluoride and ytterbium fluoride. Preferably, the nano-scale particles are particles of silica, alumina, titania, prepared by flame pyrolysis.

The average particle size of the nano-scale particles is preferable 1 to 50 nm, and more preferably 3 to 40 nm. The average particle size of the nano-scale particles can be measured by taking electron micrographs of these nano-scale particles and calculating the average value of the diameters of the 100 randomly-selected nano-scale particles. It is desirable that the inorganic nano-scale particles be subjected previously to surface treatment with a surface treating agent to improve the affinity between the inorganic filler and the polymerizable composition of the present invention, and to increase the chemical bonding between the inorganic filler and the polymerizable composition so as to enhance the mechanical strength of the cured product.

The total amount of the particulate filler is preferably 0.1 to 500 parts by weight per 100 parts by weight of the polymerizable composition, more preferably 75 to 350 parts by weight, and particularly preferably 100 to 300 parts by weight. The amount of the nano-scale particles is preferably 0.1 to 50 parts by weight per 100 parts by weight of the polymerizable composition, more preferably 1 to 40 parts by weight, and particularly preferably 3 to 30 parts by weight.

According to a preferred embodiment, the dental composition further comprises a solvent. The solvent may be water and/or an organic solvent. The organic solvent can be used alone or two or more types thereof can be used in suitable combination. Examples of the organic solvent include methanol, ethanol, 1-propanol, 2-propanol, 1-butanol, 2-methyl-2-propanol, acetone, methyl ethyl ketone, tetrahydrofuran, diethyl ether, diisopropyl ether, hexane, toluene, chloroform, ethyl acetate, and butyl acetate
The amount of the solvent is preferably 1 to 1000 parts by weight, and more preferably 5 to 200 parts by weight, with respect to 100 parts by weight of the total amount of polymerizable matrix containing polymerizable monomers.

The dental composition of the present invention may further contain a stabilizer, a pH adjuster, an ultraviolet absorber, an antioxidant, a polymerization inhibitor, a colorant, an antibacterial agent, an X-ray contrast agent, a thickening agent, a fluorescent agent.

A stabilizer may be selected form substituted and/or unsubstituted hydroxyaromatics (e.g. butylated hydroxytoluene (BHT), hydroquinone, hydroquinone monomethyl ether (MEHQ), 3,5-di-tert-butyl-4-hydroxyanisole(2,6-di-tert-butyl-4-ethoxyphenol), 2,6-di-tert-butyl-4-(dimethylamino)methylphenol or 2,5-di-tert-butyl hydroquinone, 2-(2'-hydroxy-5'-methylphenyl)-2H-benzotriazole, 2-(2'-hydroxy-5'-t-octylphenyl)-2H-benzotriazole, 2-hydroxy-4-methoxybenzophenone (UV-9), 2-(2'-hydroxy-4',6'-di-tert-pentylphenyl)-2H-benzotriazole, 2-hydroxy-4-n-octoxybenzophenone, 2-(2'-hydroxy-5'-methacryloxyethylphenyl)-2H-benzotriazole, phenothiazine, and HALS (hindered amine light stabilizers).

The dental composition of the present invention may further contain a fluorine ion sustained-releasable filler, such as sodium fluoride, calcium fluoride, fluoroaluminosilicate glass, or sodium monofluorophosphate.

The dental composition may contain an antimicrobial agent. The antimicrobial agent may be a surfactant having an antibacterial activity, such as 12-(meth)acryloyloxydodecylpyridinium bromide or cetylpyridinium chloride.

According to a preferred embodiment, the dental composition is stable at 50 °C for at least 30 days.

The present invention also provides a use of a polymerization initiator system containing
(a) an alpha-diketone photoinitiator compound having a light absorption maximum in the range from 300 to 500 nm; and
(b) a coinitiator compound of the following formula (I):

   A-H (I)

   wherein A is a tetravalent moiety of the following formula (II)

   R¹R²R³X- (II)

   wherein
   - X: represents Si, Ge, or Sn and
   - R¹: represents a hydrogen atom, an organic moiety or a different moiety A;
   - R² and R³: which are independent from each other represent an organic moiety,
for the preparation of a dental composition selected from dental adhesives containing monomers dissolved in one or more solvents and dental composites containing monomers wherein one or more particulate fillers are dispersed.

In the use according to the present invention, the polymerization initiator system preferably further comprises one or more compounds selected from the following group:
(1) a iodonium compound of the following formula (III):

   R⁴-I⁺-R⁵ Y⁻ (III)

   wherein
   - R⁴ and R⁵: which are independent from each other represent an organic moiety, and
   - Y⁻: is an anion;
(2) a sulfonium compound of the following formula (IV):

   R⁶R⁷R⁸S⁺Y⁻ (IV)

   wherein
   - R⁶, R⁷ and R⁸: which are independent from each other, represent an organic moiety or wherein any two of R⁶, R⁷ and R⁸ form a cyclic structure together with the sulfur atom to which they are bound, and
   - Y⁻: is an anion;
(3) a phosphonium compound of the following formula (V):

   R⁹R¹⁰R¹¹P⁺Y⁻ (V)

   wherein
   - R⁹, R¹⁰ and R¹¹: which are independent from each other, represent an organic moiety, and
   - Y⁻: is an anion; and
(4) a pyridinium salt.

The invention will now be further illustrated based on the following non-imitating examples.

### Examples

### Example 1 - AG 18-188-1

4.4397 g (8.6713 mmol) 2,2-bis[4-[2-hydroxy-3-methacryloyloxypropoxy)phenyl]propane (Bis-GMA), 1.0640 g (3.7163 mmol) triethylene glycol dimethacrylate (TGDMA), 0.0206 g (0.1239 mmol) camphorquinone (CQ), 0.0453 g (0.1487) triphenylgermanium hydride (TPGeH), 0.0392 g (0.1239 mmol) diphenyliodonium chloride (DPI) and 0.0041 g (0.0128 mmol) 2,6-di-tert-butyl-p-cresol were mixed homogeneously. Polymerization enthalpy measured with the DSC 7 (Perkin Elmer) is summarized in Table 1.

### Comparative Example 1 - AG 18-188-2

4.4693 g (8.7291 mmol) 2,2-bis[4-[2-hydroxy-3-methacryloyloxypropoxy)phenyl]propane (Bis-GMA), 1.0711 g (3.7410 mmol) triethylene glycol dimethacrylate (TGDMA), 0.0207 g (0.1247 mmol) camphorquinone (CQ), 0.0298 g (0.1496) 4-(dimethylamino) benzoic acid ethylester (DMABE), 0.0392 g (0.1239 mmol) diphenyliodonium chloride (DPI) and 0.0041 g (0.0128 mmol) 2,6-di-tert-butyl-p-cresol were mixed homogeneously. Polymerization enthalpy measured with the DSC 7 (Perkin Elmer) is summarized in Table 1.

**Table 1: Polymerization enthalpy of a Bis-GMA/TGDMA (70/30 w/w) composition.**

| | CQ [mol-%] | DMABE [mol-%] | TPGeH [mol-%] | DPI [mol-%] | Δ_{R}H [kJ/mol] |
|---|---|---|---|---|---|
| Example 1 | 0.9680 | - | 1.1616 | 0.9680 | 57.0 |
| Comparative Example 1 | 0.9680 | 1.1616 | - | 0.9680 | 46.7 |

The DSC measurements show a 22 % higher polymerization enthalpy of the in germanium hydride basing initiator (Example 1) compared to the CQ/amine system (Comparative Example 1).

### Comparative Example 2

| | Molar mass g/mol | amount g | mol mmol | content Gew.-% | content mol-% |
|---|---|---|---|---|---|
| Bis-GMA | 512,00 | 39,980 | 78,086 | 39,307 | 31,537 |
| TGDMA | 286,32 | 24,987 | 87,271 | 24,567 | 35,247 |
| UDMA | 470,56 | 34,982 | 74,342 | 34,393 | 30,025 |
| BHT | 220,36 | 0,050 | 0,228 | 0,049 | 0,092 |
| CQ | 166,22 | 0,398 | 2,397 | 0,392 | 0,968 |
| **DMABE** | **193,25** | **0,556** | **2,876** | **0,546** | **1,162** |
| TPGeH | 304,92 | 0,000 | 0,000 | 0,000 | 0,000 |
| DPI | 316,57 | 0,759 | 2,397 | 0,746 | 0,968 |
| Sum | | 101,713 | 247,597 | 100,000 | 100,000 |

### Example 2

### 1/ Compounds:

Triphenylgermanium hydride (Ph₃GeH), diphenyl iodonium hexafluorophosphate (Ph₂I⁺) and camphorquinone (CQ) were obtained from Sigma-Aldrich. Ethyldiethylaminobenzoate (EDB - Esacure EDB from Lamberti) was chosen as a reference amine co-initiator.

Bisphenol A-glycidyl methacrylate (Bis-GMA) and triethyleneglycol dimethacrylate (TEGDMA) were used with the highest purity available. A blend Bis-GMA/TEGDMA (70%/30% w/w) was used as benchmark matrix for dental material photopolymerizations.

### 2/ Irradiation Sources:

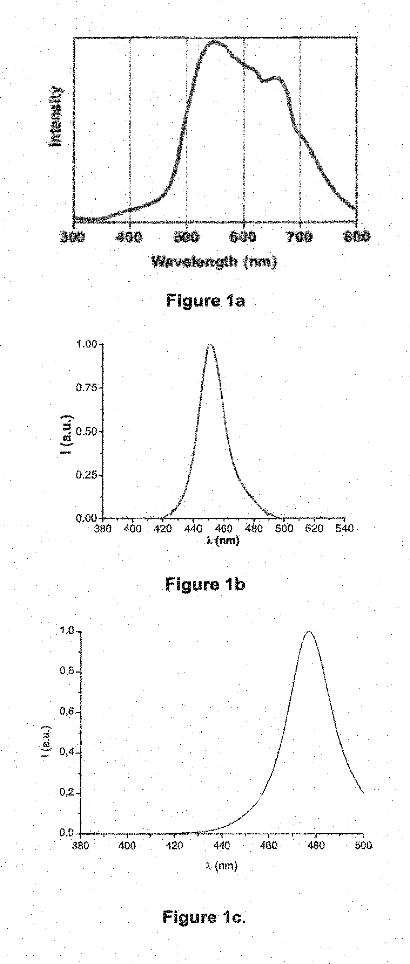

Different visible lights were used for the irradiation of samples: i) polychromatic light from a halogen lamp (Fiber-Lite, DC-950; incident light intensity: -12 mW cm⁻² in the 370-800 nm range), ii) blue LED at 455 nm (M455-L3 - ThorLabs; ~80 mW cm⁻²), and iii) blue LED at 477 nm (Dentsply SmartLite Focus, ~80 mW cm⁻²). The emission spectra of these irradiation devices are given in Figure 1a-c. Figure 1a shows the emission spectrum of the halogen lamp. Figure 1b shows the emission spectrum of blue LED centered at 455 nm. Figure 1c shows the emission spectrum of blue LED centered at 477 nm (SmartLite Focus from Dentsply).

### 3/ Photopolymerization Experiments:

For photopolymerization experiments, the conditions are given in the figure captions. The photosensitive formulations were deposited on a BaF₂ pellet *under air or in laminate* (25 □m thick) for irradiation with different lights. The evolution of the double bond content of blend of Bis-GMA/TEGDMA was continuously followed by real time FTIR spectroscopy (JASCO FTIR 4100)[1-2] at about 1630 cm⁻¹. The evolution of the Ge-H content can be also followed at 2030 cm⁻¹ for the Ph₃GeH based formulations.

### 4/ ESR Spin Trapping (ESR-ST) Experiment

ESR-ST experiment was carried out using an X-Band spectrometer (MS 400 Magnettech). The radicals were generated at room temperature upon the SmartLite Focus exposure under N₂ and trapped by phenyl-*N*-*tert*-butylnitrone (PBN) according to a procedure[3] described elsewhere in detail. The ESR spectra simulations were carried out with the WINSIM software.

### Results and Discussions:

### 1/ The polymerization initiating ability of the CQ/Ph₃GeH/Ar₂I⁺ system:

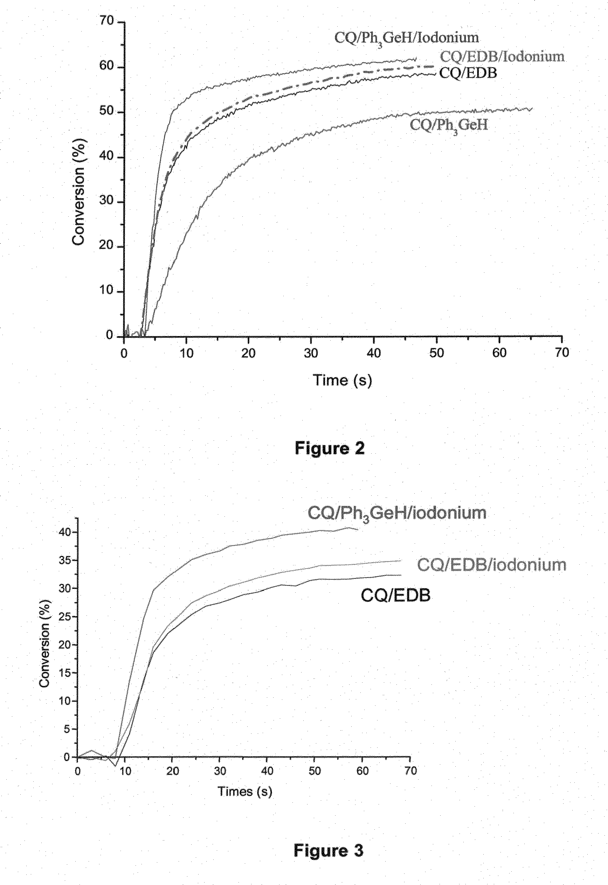

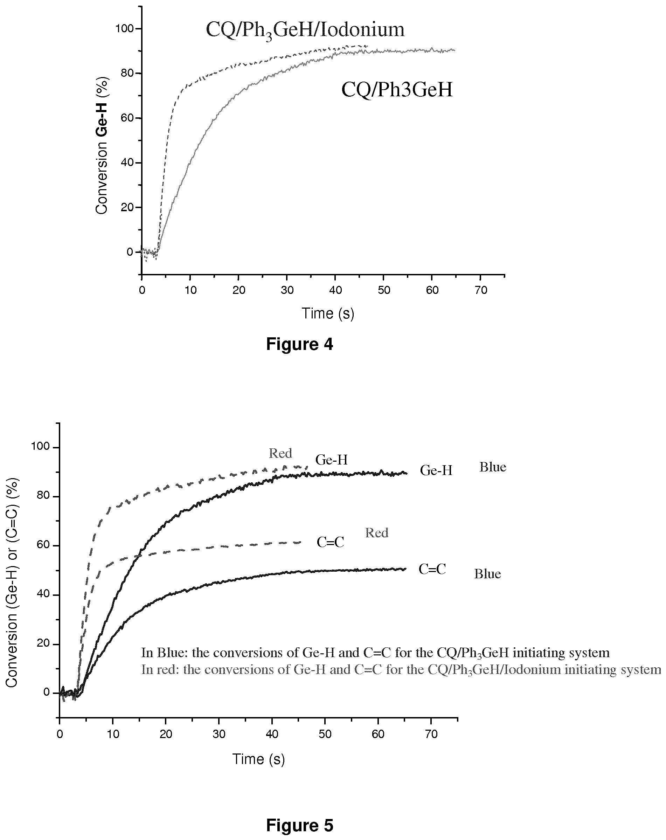

The CQ/Ph₃GeH system exhibits excellent efficiency upon blue LED irradiation (Figure 2). However, for the polymerization of a blend of Bis-GMA/TEGDMA, EDB is found as a better co-initiator than Ph₃GeH i.e. in laminate, a better polymerization profile is obtained for CQ/EDB than for CQ/Ph₃GeH (Figure 2). In presence of an iodonium salt, a completely different situation is found. This shows that the chemical mechanisms are different in presence of Ar₂I⁺. Indeed, interestingly, the CQ/Ph₃GeH/Ar₂I+ system can very efficiently initiate the methacrylates (i.e. Bis-GMA/TEGDMA blend (70%/30%, w/w)) under air or in laminate under the dental LED (SmartLite Focus), the LED@455 nm or the halogen lamp (Figures 2 and 3). The polymerization profiles (polymerization rates and final conversion) are clearly better for the new proposed CQ/Ph₃GeH/Ar₂I⁺ than for the CQ/EDB and CQ/EDB/Ar₂I⁺ systems (Figure 2 for polymerization in laminate and Figure 3 for polymerization under air). The conversion of the Ge-H content can also be specifically followed in the course of these photopolymerization experiments (see the experimental part). It can be observed that the methacrylate and the Ge-H contents exhibit very similar conversion profiles showing that the hydrogen abstraction from Ge-H governs the polymerization of methacrylates (Figures 4 and 5).

Figure 2 shows the polymerization profiles of a dental resin in laminate upon a "SmartLite Focus" exposure for different initiating systems; CQ/co-initiator 3%/2% w/w or CQ/co-initiator/ Ar₂I⁺ 3%/2%/2% w/w.

Figure 3 shows the polymerization profiles of a dental resin under air upon a "SmartLite Focus" exposure for different initiating systems; CQ/co-initiator 3%/2% w/w or CQ/co-initiator/ Ar₂I⁺ 3%/2%/2% w/w.

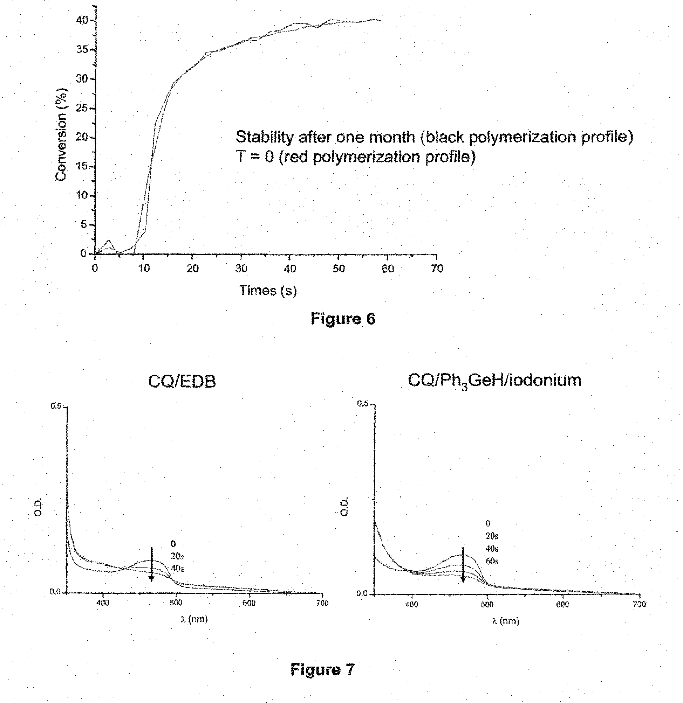

The stability of the CQ/Ph₃GeH/Ar₂I⁺ based formulation is excellent i.e. a similar polymerization profiles is obtained after one month (storage at RT; without inert gas) (Figure 6).

Figure 4 shows conversions of the Ge-H function in the course of the polymerization of the dental resin in laminate upon a "SmartLite Focus" exposure for different initiating systems; CQ/co-initiator 3%/2% w/w or CQ/co-initiator/ Ar₂I⁺ 3%/2%/2% w/w.

Figure 5 shows conversions of the Ge-H and the methacrylate functions in the course of the polymerization of the dental resin in laminate upon a "SmartLite Focus" exposure for different initiating systems; CQ/co-initiator 3%/2% w/w or CQ/co-initiator/ Ar₂I⁺ 3%/2%/2% w/w.

Figure 6 shows polymerization profiles of a dental resin under air upon a "SmartLite Focus" exposure for the CQ/Ph3GeH/iodonium initiating system (3%/2%/2% w/w).

The bleaching of the CQ/Ph₃GeH/Ar₂I⁺ based formulation in the course of the photopolymerization can be followed by UV-visible spectroscopy and is found excellent (the bleaching rate is similar than for the CQ/EDB initiating system) (Figure 7).

Figure 7 shows bleaching of the formulation in the course of the photopolymerization upon a Smarlite Focus irradiation (CQ/co-initiator 3%/2% w/w or CQ/co-initiator/Ar₂I⁺ 3%/2%/2% w/w).

### 2/ The chemical mechanisms:

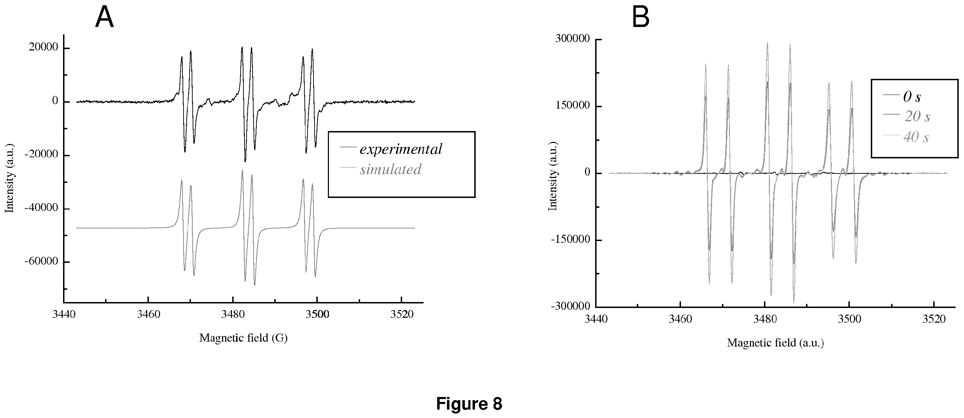

A chemical mechanism is given in (1-4), which is based on ESR-spin trapping experiments of i) aryl radicals obtained by photolysis of a CQ/Ar₂I⁺ solution (reactions 2-3; Figure 8A) as well as of ii) germyl radicals (Ph₃Ge^{•}) obtained by irradiation of a CQ/Ph₃GeH/Ar₂I⁺ solution (reaction 4 is a Ge-H hydrogen abstraction process; Figure 8B). Figure 8A shows in aryl radicals observed by ESR for the irradiation of a CQ/iodonium solution in tert-butylbenzene; Figure 8B shows that only germyl radicals are observed by ESR after irradiation of a CQ/Ph₃GeH/iodonium solution in tert-butylbenzene.

The high reactivity of the three-component system (CQ/Ph₃GeH/Ar₂I⁺) is ascribed to the very high rate constants of addition of germyl radicals onto (meth)acrylate double bond [1]; these latter rate constants being much higher than for the aminoalkyl radical derived from EDB.[1]

CQ → ¹CQ(hv) and ¹CQ → ³CQ (1)

³CQ + Ar₂I⁺ → CQ^{•+} + Ar₂I^{•} (2)

Ar₂I^{•} → Ar^{•} + Ph-I (3)

Ar^{•} + Ph₃GeH → ArH + Ph₃Ge^{•} (4)

### References:

[1] Fouassier, J.P.; Lalevée, J., Photoinitiators for Polymer Synthesis-Scope, Reactivity, and Efficiency. Wiley-VCH Verlag GmbH & Co. KGaA: Weinheim, 2012.
[2] a) Tehfe, M.-A.; Lalevée, J.; Telitel, S.; Sun, J.; Zhao, J.; Graff, B.; Morlet-Savary, F.; Fouassier, J.-P. Polymer 2012, 53, 2803-2808; b) Tehfe, M. A.; Lalevée, J.; Morlet-Savary, F.; Graff, B.; Blanchard, N.; Fouassier, J. P. Macromolecules 2012, 45, 1746-1752.
[3] Lalevée, J.; Blanchard, N.; Tehfe, M. A.; Peter, M.; Morlet-Savary, F.; Gigmes, D.; Fouassier, J. P. Polym. Chem. 2011, 2, 1986-1991.

## Claims

1. Dental composition comprising
(i) 5 to 80 percent by weight based on the total weight of the composition of a polymerizable matrix containing polymerizable monomers;
(ii) a polymerization initiator system containing
(a) an alpha-diketone photoinitiator compound having a light absorption maximum in the range from 300 to 500 nm; and
(b) a coinitiator compound of the following formula (I):
A-H (I)
wherein A is a moiety of the following formula (II)
R¹R²R³X (II)
wherein
X represents Si, Ge, or Sn and
R¹ represents a hydrogen atom, an organic moiety or a different moiety A;
R² and R³ which are independent from each other, represent an organic moiety,
wherein the dental composition is selected from dental adhesives containing monomers dissolved in one or more solvents and dental composites containing monomers wherein one or more particulate fillers are dispersed.

2. The dental composition according to claim 1, wherein the polymerization initiator system further comprises one or more compounds selected from the following group:
(1) an iodonium compound of the following formula (III):
R⁴-I⁺-R⁵ Y⁻ (III)
wherein
R⁴ and R⁵ which are independent from each other represent an organic moiety, and
Y⁻ is an anion;
(2) a sulfonium compound of the following formula (IV):
R⁶R⁷R⁸S⁺Y⁻ (IV)
wherein
R⁶, R⁷ and R⁸ which are independent from each other, represent an organic moiety or wherein any two of R⁶, R⁷ and R⁸ form a cyclic structure together with the sulfur atom to which they are bound, and
Y⁻ is an anion;
(3) a phosphonium compound of the following formula (V):
R⁹R¹⁰R¹¹P⁺Y⁻ (V)
wherein
R⁹, R¹⁰ and R¹¹ which are independent from each other, represent an organic moiety, and
Y⁻ is an anion; and
(4) a pyridinium salt.

3. The dental composition according to any one of claims 1 or 2, which further comprises a particulate filler.

4. The dental composition according to any one of the preceding claims which further comprises a solvent.

5. The dental composition according to any one of the preceding claims,
wherein the 1,2-diketone photoinitiator compound is selected from camphor quinone and 1,2-diphenylethane-1,2-dione.

6. The dental composition according to any one of the preceding claims,
wherein R¹, R², and R³ in the coinitiator compound of formula (I) are the same and represent an aliphatic, an aromatic or an alicyclic hydrocarbon group.

7. The dental composition according to any one of the preceding claims,
wherein the coinitiator compound of formula (I) is a compound of the following formula:

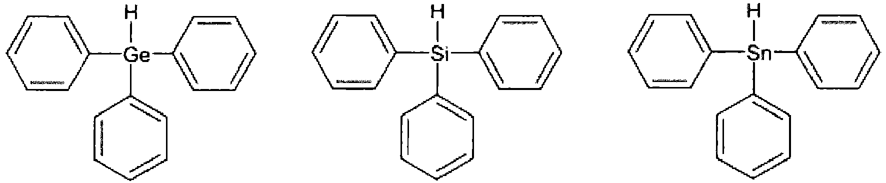

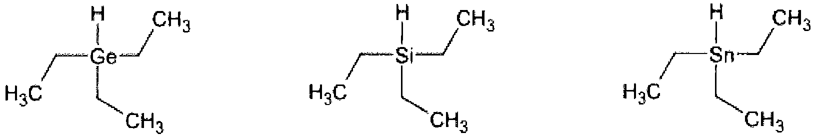

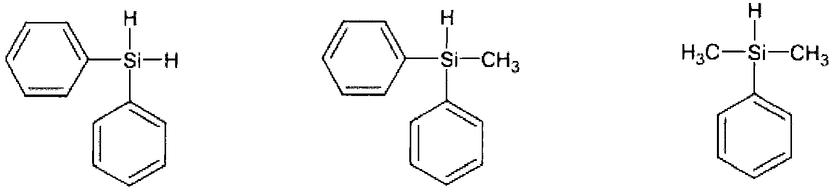

8. The dental composition according to any one of the preceding claims, which is stable at 50 °C for at least 30 days.

9. The dental composition according to any one of the preceding claims, which contains the polymerization initiator system in an amount from 0.01 to 10 weight percent based on the total weight of the composition.

10. The dental composition according to any one of the preceding claims wherein the polymerizable matrix contains the alpha-diketone photoinitiator in an amount from 0.05 to 5 mole percent.

11. The dental composition according to any one of the preceding claims wherein the polymerizable matrix contains the coinitiator compound in an amount from 0.05 to 5 percent by weight based on the total weight of the composition.

12. The dental composition according to any one of the preceding claims wherein the polymerizable marix contains the diphenyl iodonium compound in an amount from 0.001 to 2 percent by weight based on the total weight of the composition.

13. The dental composition according to any one of the preceding claims which is a dental adhesive or a dental composite.

14. Use of a polymerization initiator system containing
(a) an alpha-diketone photoinitiator compound having a light absorption maximum in the range from 300 to 500 nm; and
(b) a coinitiator compound of the following formula (I):
A-H (I)
wherein A is a tetravalent moiety of the following formula (II)
R¹R²R³X- (II)
wherein
X represents Si, Ge, or Sn and
R¹ represents a hydrogen atom, an organic moiety or a different moiety A;
R² and R³ which are independent from each other represent an organic moiety,
for the preparation of a dental composition selected from dental adhesives containing monomers dissolved in one or more solvents and dental composites containing monomers wherein one or more particulate fillers are dispersed.

15. The use according to claim 14, wherein the polymerization initiator system further comprises one or more compounds selected from the following group:
(1) an iodonium compound of the following formula (III):
R⁴-I⁺-R⁵ Y⁻ (III)
wherein
R⁴ and R⁵ which are independent from each other represent an organic moiety, and
Y⁻ is an anion;
(2) a sulfonium compound of the following formula (IV):
R⁶R⁷R⁸S⁺Y⁻ (IV)
wherein
R⁶, R⁷ and R⁸ which are independent from each other, represent an organic moiety or wherein any two of R⁶, R⁷ and R⁸ form a cyclic structure together with the sulfur atom to which they are bound, and
Y⁻ is an anion;
(3) a phosphonium compound of the following formula (V):
R⁹R¹⁰R¹¹P⁺Y⁻ (V)
wherein
R⁹, R¹⁰ and R¹¹ which are independent from each other, represent an organic moiety, and
Y⁻ is an anion; and
(4) a pyridinium salt.

## Patentansprüche

1. Dentale Zusammensetzung, umfassend
(i) 5 bis 80 Gewichtsprozent, bezogen auf das Gesamtgewicht der Zusammensetzung, einer polymerisierbaren Matrix, die polymerisierbare Monomere enthält;
(ii) ein Polymerisationsinitiatorsystem, enthaltend
(a) eine alpha-Diketon-Photoinitiatorverbindung mit einem Lichtabsorptionsmaximum im Bereich von 300 bis 500 nm; und
(b) eine Coinitiatorverbindung der folgenden Formel (I):
A-H (I)
wobei A eine Einheit der folgenden Formel (II) ist
R¹R²R³X (II)
wobei
X Si, Ge oder Sn darstellt; und
R¹ ein Wasserstoffatom, eine organische Einheit oder eine andere Einheit A darstellt;
R² und R³
unabhängig voneinander eine organische Einheit darstellen,
wobei die dentale Zusammensetzung ausgewählt ist aus Dentalklebstoffen, die in einem oder mehreren Lösungsmitteln gelöste Monomere enthalten, und Dentalverbundwerkstoffen, die Monomere enthalten, in denen ein oder mehrere teilchenförmige Füllstoffe dispergiert sind.

2. Dentale Zusammensetzung nach Anspruch 1, wobei das Polymerisationsinitiatorsystem ferner eine oder mehrere Verbindungen umfasst, ausgewählt aus der folgenden Gruppe:
(1) eine lodoniumverbindung der folgenden Formel (III):
R^{a}-I⁺-R⁵ Y⁻ (III)
wobei
R⁴ und R⁵
unabhängig voneinander eine organische Einheit darstellen, und
Y⁻ ein Anion ist;
(2) eine Sulfoniumverbindung der folgenden Formel (IV):
R⁶R⁷R⁸S⁺Y⁻ (IV)
wobei
R⁶, R⁷ und R⁸
unabhängig voneinander eine organische Einheit darstellen oder wobei zwei von R⁶, R⁷ und R⁸ zusammen mit dem Schwefelatom, an das sie gebunden sind, eine cyclische Struktur bilden, und
Y⁻ ein Anion ist;
(3) eine Phosphoniumverbindung der folgenden Formel (V):
R⁹R¹⁰R¹¹P⁺Y⁻ (V)
wobei
R⁹, R¹⁰ und R¹¹
unabhängig voneinander eine organische Einheit darstellen, und
Y⁻ ein Anion ist; und
(4) ein Pyridiniumsalz.

3. Dentale Zusammensetzung nach einem der Ansprüche 1 oder 2, die ferner einen teilchenförmigen Füllstoff umfasst.

4. Dentale Zusammensetzung nach einem der vorhergehenden Ansprüche, die ferner ein Lösungsmittel umfasst.

5. Dentale Zusammensetzung nach einem der vorhergehenden Ansprüche, wobei die 1,2-Diketon-Photoinitiatorverbindung ausgewählt ist aus Campherchinon und 1,2-Diphenylethan-1,2-dion.

6. Dentale Zusammensetzung nach einem der vorhergehenden Ansprüche, wobei R¹, R² und R³ in der Coinitiatorverbindung der Formel (I) gleich sind und eine aliphatische, eine aromatische oder eine alicyclische Kohlenwasserstoffgruppe darstellen.

7. Dentale Zusammensetzung nach einem der vorhergehenden Ansprüche, wobei die Coinitiatorverbindung der Formel (I) eine Verbindung der folgenden Formel ist:

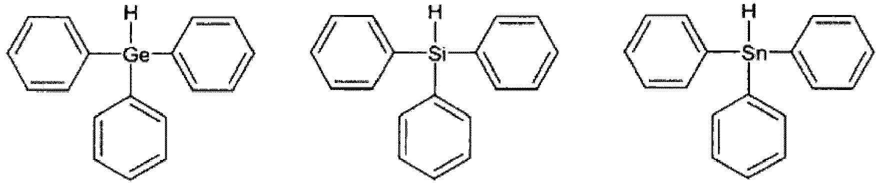

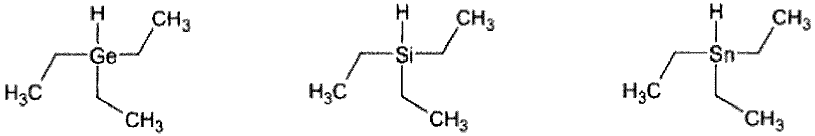

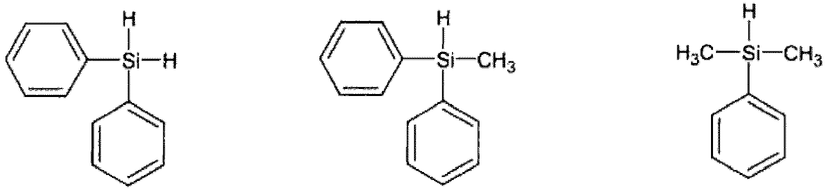

8. Dentale Zusammensetzung nach einem der vorhergehenden Ansprüche, die mindestens 30 Tage bei 50 °C stabil ist.

9. Dentale Zusammensetzung nach einem der vorhergehenden Ansprüche, die das Polymerisationsinitiatorsystem in einer Menge von 0,01 bis 10 Gewichtsprozent, bezogen auf das Gesamtgewicht der Zusammensetzung, enthält.

10. Dentale Zusammensetzung nach einem der vorhergehenden Ansprüche, wobei die polymerisierbare Matrix den alpha-Diketon-Photoinitiator in einer Menge von 0,05 bis 5 Mol-% enthält.

11. Dentale Zusammensetzung nach einem der vorhergehenden Ansprüche, wobei die polymerisierbare Matrix die Coinitiatorverbindung in einer Menge von 0,05 bis 5 Gewichtsprozent, bezogen auf das Gesamtgewicht der Zusammensetzung, enthält.

12. Dentale Zusammensetzung nach einem der vorhergehenden Ansprüche, wobei die polymerisierbare Matrix die Diphenyliodoniumverbindung in einer Menge von 0,001 bis 2 Gewichtsprozent, bezogen auf das Gesamtgewicht der Zusammensetzung, enthält.

13. Dentale Zusammensetzung nach einem der vorhergehenden Ansprüche, die ein Dentalkleber oder ein Dentalverbundstoff ist.

14. Verwendung eines Polymerisationsinitiatorsystems, enthaltend
(a) eine alpha-Diketon-Photoinitiatorverbindung mit einem Lichtabsorptionsmaximum im Bereich von 300 bis 500 nm; und
(b) eine Coinitiatorverbindung der folgenden Formel (I):
A-H (I)
wobei A eine vierwertige Einheit der folgenden Formel (II) ist
R¹R²R³X- (II)
wobei
X Si, Ge oder Sn darstellt; und
R¹ ein Wasserstoffatom, eine organische Einheit oder eine andere Einheit A darstellt;
R² und R³
unabhängig voneinander eine organische Einheit darstellen,
zur Herstellung einer dentalen Zusammensetzung, die ausgewählt ist aus Dentalklebstoffen, die in einem oder mehreren Lösungsmitteln gelöste Monomere enthalten, und Dentalverbundwerkstoffen, die Monomere enthalten, in denen ein oder mehrere teilchenförmige Füllstoffe dispergiert sind.

15. Verwendung nach Anspruch 14, wobei das Polymerisationsinitiatorsystem ferner eine oder mehrere Verbindungen umfasst, ausgewählt aus der folgenden Gruppe:
(1) eine lodoniumverbindung der folgenden Formel (III):
R^{a}-I⁺-R⁵Y⁻ (III)
wobei
R⁴ und R⁵
unabhängig voneinander eine organische Einheit darstellen, und
Y⁻ ein Anion ist;
(2) eine Sulfoniumverbindung der folgenden Formel (IV):
R⁶R⁷R⁸S⁺y⁻ (IV)
wobei
R⁶, R⁷ und R⁸
unabhängig voneinander eine organische Einheit darstellen oder wobei zwei von R⁶, R⁷ und R⁸ zusammen mit dem Schwefelatom, an das sie gebunden sind, eine cyclische Struktur bilden, und
Y⁻ ein Anion ist;
(3) eine Phosphoniumverbindung der folgenden Formel (V):
R⁹R¹⁰R¹¹P⁺Y⁻ (V)
wobei
R⁹, R¹⁰ und R¹¹
unabhängig voneinander eine organische Einheit darstellen, und Y- ein Anion ist; und
(4) ein Pyridiniumsalz.

## Revendications

1. Composition dentaire comprenant
(i) de 5 à 80 % en poids sur la base du poids total de la composition d'une matrice polymérisable contenant des monomères polymérisables ;
(ii) un système amorceur de polymérisation contenant
(a) un composé photo-amorceur alpha-dicétone ayant un maximum d'absorption de la lumière dans la plage de 300 à 500 nm ; et
(b) un composé co-amorceur de formule suivante (I) :
A-H (I)
dans laquelle A est un fragment de formule suivante (II)
R¹R²R³X (II)
dans laquelle
X représente Si, Ge ou Sn et
R¹ représente un atome d'hydrogène, un fragment organique ou un fragment différent A;
R² et R³
qui sont indépendants l'un de l'autre, représentent un fragment organique,
la composition dentaire étant sélectionnée parmi des adhésifs dentaires contenant des monomères dissous dans un ou plusieurs solvants et des composites dentaires contenant des monomères dans lesquels une ou plusieurs charges particulaires sont dispersées.

2. Composition dentaire selon la revendication 1, dans laquelle le système amorceur de polymérisation comprend en outre un ou plusieurs composés sélectionnés dans le groupe suivant :
(1) un composé iodonium de formule suivante (III) :
R^{a}-I⁺-R⁵Y⁻ (III)
dans laquelle
R⁴ et R⁵
qui sont indépendants l'un de l'autre représentent un fragment organique et
Y⁻ est un anion ;
(2) un composé sulfonium de formule suivante (IV) :
R⁶R⁷R⁸S⁺Y⁻ (IV)
dans laquelle
R⁶, R⁷ et R⁸
qui sont indépendants l'un de l'autre, représentent un fragment organique ou dans laquelle deux quelconques de R⁶, R⁷ et R⁸ forment une structure cyclique conjointement avec l'atome de soufre auquel ils sont liés et
Y⁻ est un anion ;
(3) un composé phosphonium de formule suivante (V) :
R⁹R¹⁰R¹¹P⁺Y⁻ (V)
dans laquelle
R⁹, R¹⁰ et R¹¹
qui sont indépendants l'un de l'autre, représentent un fragment organique et
Y⁻ est un anion ; et
(4) un sel de pyridinium.

3. Composition dentaire selon l'une quelconque des revendications 1 ou 2, qui comprend en outre une charge particulaire.

4. Composition dentaire selon l'une quelconque des revendications précédentes, qui comprend en outre un solvant.

5. Composition dentaire selon l'une quelconque des revendications précédentes, dans laquelle le composé photo-amorceur 1,2-dicétone est sélectionné parmi la camphoquinone et la 1,2-diphényléthane-1,2-dione.

6. Composition dentaire selon l'une quelconque des revendications précédentes, dans laquelle R¹, R² et R³ dans le composé co-amorceur de formule (I) sont identiques et représentent un groupe hydrocarboné aliphatique, aromatique ou alicyclique.

7. Composition dentaire selon l'une quelconque des revendications précédentes, dans laquelle le composé co-amorceur de formule (I) est un composé de formule suivante :

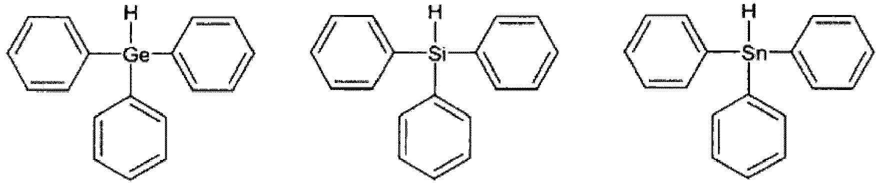

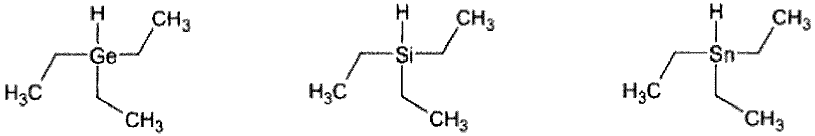

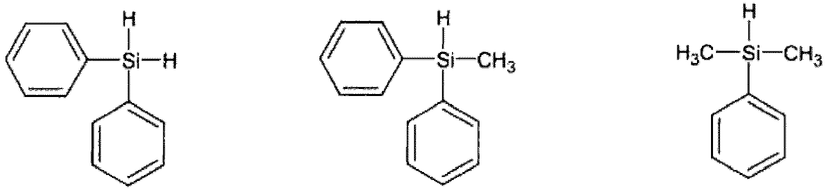

8. Composition dentaire selon l'une quelconque des revendications précédentes, qui est stable à 50 °C pendant au moins 30 jours.

9. Composition dentaire selon l'une quelconque des revendications précédentes, qui contient le système amorceur de polymérisation en une quantité de 0,01 à 10 % en poids sur la base du poids total de la composition.

10. Composition dentaire selon l'une quelconque des revendications précédentes, dans laquelle la matrice polymérisable contient le photo-amorceur alpha-dicétone en une quantité de 0,05 à 5 % en moles.

11. Composition dentaire selon l'une quelconque des revendications précédentes, dans laquelle la matrice polymérisable contient le composé co-amorceur en une quantité de 0,05 à 5 % en poids sur la base du poids total de la composition.

12. Composition dentaire selon l'une quelconque des revendications précédentes, dans laquelle la matrice polymérisable contient le composé diphényliodonium en une quantité de 0,001 à 2 % en poids sur la base du poids total de la composition.

13. Composition dentaire selon l'une quelconque des revendications précédentes, qui est un adhésif dentaire ou un composite dentaire.

14. Utilisation d'un système amorceur de polymérisation contenant
(a) un composé photo-amorceur alpha-dicétone ayant un maximum d'absorption de la lumière dans la plage de 300 à 500 nm ; et
(b) un composé co-amorceur de formule suivante (I) :
A-H (I)
dans laquelle A est un fragment tétravalent de formule suivante (II)
R¹R²R³X- (II)
dans laquelle
X représente Si, Ge ou Sn et
R¹ représente un atome d'hydrogène, un fragment organique ou un fragment différent A ; R² et R³
qui sont indépendants l'un de l'autre représentent un fragment organique
pour la préparation d'une composition dentaire sélectionnée parmi des adhésifs dentaires contenant des monomères dissous dans un ou plusieurs solvants et des composites dentaires contenant des monomères dans lesquels une ou plusieurs charges particulaires sont dispersées.

15. Utilisation selon la revendication 14, dans laquelle le système amorceur de polymérisation comprend en outre un ou plusieurs composés sélectionnés dans le groupe suivant :
(1) un composé iodonium de formule suivante (III) :
R^{a}-I⁺-R⁵Y⁻ (III)
dans laquelle
R⁴ et R⁵
qui sont indépendants l'un de l'autre représentent un fragment organique et
Y⁻ est un anion ;
(2) un composé sulfonium de formule suivante (IV) :
R⁶R⁷R⁸S⁺Y⁻ (IV)
dans laquelle
R⁶, R⁷ et R⁸
qui sont indépendants l'un de l'autre, représentent un fragment organique ou dans laquelle deux quelconques de R⁶, R⁷ et R⁸ forment une structure cyclique conjointement avec l'atome de soufre auquel ils sont liés et
Y⁻ est un anion ;
(3) un composé phosphonium de formule suivante (V) :
R⁹R¹⁰R¹¹P⁺ Y⁻ (V)
dans laquelle
R⁹, R¹⁰ et R¹¹
qui sont indépendants l'un de l'autre, représentent un fragment organique et
Y⁻ est un anion ; et
(4) un sel de pyridinium.
